# EUROPÄISCHE PATENTANMELDUNG

(11) **EP 4 555 960 A1**
(43) Veröffentlichungstag der Anmeldung: **21.05.2025**
(21) Anmeldenummer: 24212762.9
(22) Anmeldetag: 13.11.2024
(51) Int. Cl.: A61B 34/20, A61B 90/20, G02B 21/00

(54) **PRIORISIERUNG MEHRERER OBJEKTE BEI ASSISTENZFUNKTIONALITÄT FÜR OPERATIONSMIKROSKOPIESYSTEM**

(30) Priorität: 15.11.2023 DE 102023131861
(71) Anmelder: Carl Zeiss Meditec AG, 07745 Jena (DE)
(72) Erfinder: PHILIPP, Markus, 73447 Oberkochen (DE); SAUR, Stefan, 73447 Oberkochen (DE); BACHER, Neal, 73447 Oberkochen (DE)
(74) Vertreter: Kraus & Lederer PartGmbB

(57) **Zusammenfassung**

Es werden Techniken im Zusammenhang mit dem Ausführen eine Assistenzfunktionalität für ein Operationsmikroskopiesystem beschrieben. Die Assistenzfunktionalität - z.B. eine Auto-Zentrierung oder eine Vermessung eines Operationsinstruments - wird im Zusammenhang mit mehreren Objekten (231, 232, 233) ausgeführt, die in einem mittels des Operationsmikroskopiesystems erfassten Bild (220) abgebildeten sind. Außerdem wird auch Priorisierungsinformation (241, 242, 243) für die Objekte (231, 232, 233) berücksichtigt, z.B. um eine Klasseneinteilung in relevante und nicht relevante Objekte vorzunehmen.

## Beschreibung

### TECHNISCHES GEBIET

Verschiedene Beispiele der Offenbarung betreffen Techniken, um eine Assistenzfunktionalität für ein Operationsmikroskopiesystem auszuführen. Verschiedene Beispiele betreffen insbesondere die Priorisierung zwischen unterschiedlichen Objekten im Zusammenhang mit einer solchen Assistenzfunktionalität.

### HINTERGRUND

Medizinische Operationsmikroskopiesysteme (auch als robotische Visualisierungssysteme oder chirurgische Visualisierungssysteme bezeichnet) mit einem robotischem Stativ zur Positionierung eines Mikroskops sind aus dem Stand der Technik bekannt, siehe z.B. DE 10 2022 100 626 A1.

Das robotische Stativ kann dabei manuell gesteuert werden. Es sind aber auch Techniken bekannt, bei denen das robotische Stativ automatisch gesteuert wird, z.B. um eine Auto-Zentrierung und/oder Auto-Fokussierung auf ein bestimmtes Objekt wie z.B. ein Operationsinstrument (auch als Operationsbesteck oder Operationswerkzeug oder Operationstool bezeichnet) zu ermöglichen. Ein Benutzerbefehl löst dabei einen Positionierungsvorgang aus, bei denen das robotische Stativ und/oder eine Objektivoptik des Mikroskops angesteuert werden. Solche Techniken sind z.B. bekannt aus US 10,456,035 B2.

Es wurde beobachtet, dass insbesondere bei komplexeren chirurgischen Szenen - z.B. mit einer Vielzahl von Operationsinstrumenten und/oder Operationsinstrumenten unterschiedlichen Typs solche Techniken aus dem Stand der Technik unerwünschte Ergebnisse liefern können. Beispielsweise erfolgt manchmal eine Positionierung auf ein falsches Operationsinstrument, das vom Operateur gar nicht gemeint war.

Um solche Nachteile zu beheben, ist es zum Beispiel aus US 10,769,443 B2 bekannt, dominante Operationsinstrumente aus einer Menge von sichtbaren Operationsinstrumenten zu erkennen. Auch solche Techniken weisen bestimmte Nachteile und Einschränkungen auf. Beispielweise wurde festgestellt, dass die dort beschriebenen Techniken für manche Operationsinstrument-Typen nicht gut funktionieren. Das bedeutet, dass je nach Operationsinstrument-Typ schlechte Ergebnisse erzielt werden. Aufgrund der großen Anzahl von verfügbaren Operationsinstrumentenschwächt dies die Akzeptanz des Systems und unerwartetes Verhalten kann auftreten.

### ZUSAMMENFASSUNG

Deshalb besteht ein Bedarf für verbesserte Techniken für Assistenzfunktionalitäten für Operationsmikroskopiesysteme. Insbesondere besteht ein Bedarf für Techniken, welche eine robuste Einstufung der Priorität von abgebildeten Objekten, auf die sich die Assistenzfunktionalität beziehen kann, ermöglichen. Es besteht ein Bedarf, um Objekte als dominant oder nicht dominant zu klassifizieren.

Diese Aufgabe wird gelöst von den Merkmalen der unabhängigen Ansprüche. Die Merkmale der abhängigen Ansprüche definieren Ausführungsformen.

Verschiedene Beispiele beruhen auf der Erkenntnis, dass Techniken zur Erkennung von dominanten Operationsinstrumenten, wie sie im Stand der Technik bekannt sind, eine geringe Robustheit gegenüber Variationen im Erscheinungsbild der Operationsinstrumente aufweisen. Dies liegt daran, dass in verschiedenen Operationsumgebungen eine besonders große Vielzahl von unterschiedlichen Operationsinstrument-Typen existiert, beispielsweise im dreistelligen Bereich. Verschiedene Beispiele beruhen auf der Erkenntnis, dass es algorithmisch schwierig möglich ist, im Rahmen einer Operationsinstrument-Typ-Klassifikation robust zwischen einer so großen Anzahl von möglichen Ergebnisklassen zu unterscheiden. Z.B. kann bei der Verwendung von maschinengelernten Klassifikationsmodellen oftmals eine "out-of-distribution" Situation auftreten, bei der das Erscheinungsbild eines spezifischen Operationsinstrument-Typs nicht im Training des maschinengelernten Klassifikationsmodells berücksichtigt wurde. Das Modell kann dann unvorhersehbare Ergebnisse liefern.

Darüber hinaus beruhen verschiedene Beispiele auf der Erkenntnis, dass Techniken zur Erkennung von dominanten Operationsinstrumenten, wie sie im Stand der Technik bekannt sind, ignorieren, dass ein bestimmter Operationsinstrument-Typ in einem Kontext dominant sein kann und in einem anderen Kontext nicht dominant sein kann. Es wurde erkannt, dass die Berücksichtigung des Kontexts bei der Priorisierung hilfreich sein kann.

Nachfolgend werden Aspekte im Zusammenhang mit einem Operationsmikroskopiesystem mit einem robotischen Stativ und einem vom robotischen Stativ getragenen Mikroskop beschrieben. Es werden insbesondere Techniken im Zusammenhang mit einer Assistenzfunktionalität zur Unterstützung eines Chirurgen beschrieben. Die Assistenzfunktionalität wird dabei in Bezug auf mindestens ein Objekt ausgeführt, welches in einem entsprechenden Bild (z.B. von einer Mikroskopkamera oder einer Umfeldkamera erfasst) abgebildet ist. Beispiele für solche Assistenzfunktionalitäten sind die Auto-Positionierung, Auto-Zentrierung, Auto-Orientierung, Auto-Zoom oder Auto-Fokus in Bezug auf ein oder mehrere Objekte; weitere Beispiele umfassen z.B. die Vermessung von ein oder mehreren Objekten.

Es werden Techniken offenbart, um relevante und nicht-relevante Objekte voneinander zu trennen. Relevante Objekte werden manchmal auch als dominante Objekte bezeichnet.

Es werden - allgemeiner formuliert - Techniken beschrieben, wie Priorisierungsinformation für die verschiedenen Objekte bestimmt werden kann. Die Assistenzfunktionalität kann dann unter Berücksichtigung der Priorisierungsinformation ausgeführt werden. Beispielsweise kann die Assistenzfunktionalität nur solche Objekte berücksichtigen, die - anhand der Priorisierungsinformation - als relevant (also mit hoher Priorität) eingestuft sind. Auch graduelle Bestimmung von entsprechende Priorisierungsinformation kann verwendet werden, so dass höher priorisierte Objekte beim Ausführen der Assistenzfunktionalität stärker berücksichtigt werden.

Die Bestimmung der Priorisierungsinformation kann, allgemein formuliert, einer Regressionsaufgabe oder einer Klassifikationsaufgabe entsprechen.

Die Bestimmung der Priorisierungsinformation erfolgt gemäß verschiedenen offenbarten Varianten basierend auf Bewegungsinformation für die verschiedenen Objekte. Die Verwendung von Bewegungsinformation weist bestimmte Vorteile in Bezug auf Referenzimplementierungen auf, bei denen eine Typ-Klassifikation der Objekte (z.B. eine Instanzsegmentierung mit zugehöriger Klassifizierung der Instanzen) erfolgen muss. Insbesondere kann eine robuste Priorisierung auch für viele verschiedene, a priori unbekannte Szenen erfolgen. Es ist beispielsweise entbehrlich, ein Klassifikationsmodell zur Bestimmung des Typs der Objekte zu parametrisieren. Beispielsweise muss kein entsprechender maschinengelerntes Klassifikationsmodell trainiert werden, was aufwendige Trainingskampagnen zum Sammeln von Bildern unterschiedlicher Typen von Objekten entbehrlich werden lässt. Die Priorisierungsinformation kann ohne eine Klassifikation und insbesondere ohne eine Instanzsegmentierung der Objekte erfolgen.

Trotzdem ist es in manchen Varianten denkbar, dass weitere Information beim Bestimmen der Priorisierungsinformation berücksichtigt wird, beispielsweise eine semantische Kontextinformation der abgebildeten Szene oder auch, ob ein bestimmtes Operationsinstrument in der linken oder rechten Hand geführt wird.

Ein Computer-implementiertes Verfahren zum Steuern eines Operationsmikroskopiesystems wird offenbart. Das Operationsmikroskopiesystem umfasst ein Stativ. Das Operationsmikroskopiesystem umfasst auch ein Mikroskop. Das Mikroskop wird vom Stativ getragen.

Zum Beispiel kann das Stativ ein robotisches Stativ sein oder ein teilweise robotische Stativ.

Das Verfahren umfasst das Ansteuern einer Kamera des Operationsmikroskopiesystems. Beispielsweise könnte eine Mikroskopkamera und eine Umfeldkamera angesteuert werden. Durch das Ansteuern der Kamera wird eine Abfolge von Bildern erhalten. Die Abfolge von Bildern kann also beispielsweise einer Zeitsequenz von Bildern, die eine Szene abbilden, entsprechen.

Das Verfahren umfasst auch das Bestimmen von Bewegungsinformation. Die Bewegungsinformation wird für jedes von zwei oder mehr in der Abfolge der Bilder abgebildeten Objekten bestimmt. Die Bewegungsinformation wird basierend auf der Abfolge von Bildern bestimmt.

Zum Bestimmen der Bewegungsinformation können zum Beispiel heuristische oder maschinengelernte Modelle eingesetzt werden. Es könnten zum Beispiel Schwellenwertvergleiche von ein oder mehreren Größen, die durch die Bewegungsinformation indiziert werden, mit ein oder mehreren vorgegebenen Schwellenwerten ausgeführt werden.

Die Bewegungsinformation kann zum Beispiel einen optischen Fluss angeben. Die Bewegungsinformation kann alternativ oder zusätzlich Aktivitätsbereiche angeben, das heißt solche Bereiche in der Abfolge der Bilder, in denen eine relativ große Veränderung des Kontrasts zwischen den Bildern der Abfolge vorliegt. Es wäre denkbar, dass die Bewegungsinformation Bewegungsmuster für die Objekte angibt. Die Bewegungsinformation kann zum Beispiel eine Bewegungsamplitude und/oder Bewegungsfrequenzen für die Objekte angeben. Auch Kombinationen aus solchen Inhalten der Bewegungsinformation wie voranstehend beschrieben sind denkbar.

Es wäre denkbar (aber nicht notwendig), dass die Bewegungsinformation mit einer Positionierung der Objekte in der Abfolge der Bilder verknüpft ist. Beispielsweise wäre es möglich, zunächst die Objekte in der Abfolge der Bilder zu lokalisieren und dann für jedes der lokalisierten Objekte eine entsprechende Bewegungsinformation zu bestimmen.

Das Verfahren umfasst weiterhin das Bestimmen von Priorisierungsinformation für die zwei oder mehr Objekte; dies erfolgt basierend auf der Bewegungsinformation. Die Priorisierungsinformation gibt beispielsweise an, welche Objekt dominant bzw. relevant sind und welche Objekte nicht dominant bzw. nicht relevant sind. Neben einer solchen binären Klassenzuweisung der verschiedenen Objekte im Rahmen der Priorisierungsinformation wäre auch eine mehrdimensionale Klassenzuweisung oder eine Regression denkbar. Z.B. könnte ein Priorisierungswert ausgegeben werden, z.B. im Bereich von 0 (=nicht relevant) bis 10 (=relevant).

Basierend auf der Priorisierungsinformation wird dann eine Assistenzfunktionalität im Zusammenhang mit den zwei oder mehr Objekten ausgeführt. Dies kann zum Beispiel bedeuten, dass solche Objekte, die eine größere (kleinere) Priorisierung aufweisen, stärker (schwächer) im Rahmen der Assistenzfunktionalität berücksichtigt werden.

Im Rahmen der Assistenzfunktionalität könnte zum Beispiel das robotische Stativ angesteuert werden, z.B. um eine Auto-Positionierung in Bezug auf einen Referenzpunkt durchzuführen, der basierend auf ein oder mehreren Objekten bestimmt ist. Im Rahmen der Assistenzfunktionalität kann das Erscheinungsbild von einem oder mehreren Objekten in einem oder mehreren Bildern, beispielsweise ein Mikroskopbild, ausgewertet werden.

Es wird einer Datenverarbeitungseinrichtung offenbart. Die Datenverarbeitungseinrichtung ist eingerichtet, um ein Operationsmikroskopiesystem zu steuern. Die Datenverarbeitungseinrichtung umfasst einem Prozessor. Der Prozessor ist eingerichtet, um Programmcode aus einem Speicher zu laden und auszuführen. Das Ausführen des Programmcodes bewirkt, dass der Prozessor das obenstehend beschriebene Verfahren zum Steuern eines Operationsmikroskopiesystems ausführt.

Es wird auch ein Operationsmikroskopiesystem offenbart, welches eine solche Datenverarbeitungseinrichtung umfasst.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### KURZE BESCHREIBUNG DER FIGUREN

FIG. 1 illustriert schematisch ein Operationsmikroskopiesystem gemäß verschiedenen Beispielen.
FIG. 2 illustriert schematisch unterschiedliche Gesichtsfelder im Zusammenhang mit einem Mikroskop und einer Umfeldkamera eines beispielhaften Operationsmikroskopiesystems.
FIG. 3 ist ein Flussdiagramm eines beispielhaften Verfahrens.
FIG. 4 ist ein Flussdiagramm eines beispielhaften Verfahrens.
FIG. 5 illustriert ein mittels einer Kamera erfassten Bilds, in welchem mehrere Operationsinstrumente sichtbar sind.
FIG. 6 entspricht FIG. 5, wobei zusätzlich Priorisierungsinformation für die verschiedenen Operationsinstrumente angezeigt ist.

### DETAILLIERTE BESCHREIBUNG

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionale Einheiten können als Hardware, Software oder eine Kombination aus Hardware und Software implementiert werden.

Nachfolgend werden Techniken im Zusammenhang mit dem Betreiben von Operationsmikroskopiesystemen beschrieben. Die beschriebenen Techniken ermöglichen es, eine Assistenzfunktionalität auszuführen, z.B. eine automatische Konfiguration von ein oder mehreren Komponenten des Operationsmikroskopiesystems. Allgemein erfolgt die Assistenzfunktionalität in Bezug auf mindestens ein Objekt aus einer Vielzahl von Objekten, die in einem mittels des Operationsmikroskopiesystems erfassten Bilds sichtbar sind.

Gemäß verschiedenen Beispielen wird Priorisierungsinformation für die Objekte bestimmt. Die Priorisierungsinformation wird dabei basierend auf Bewegungsinformation für die Objekte bestimmt.

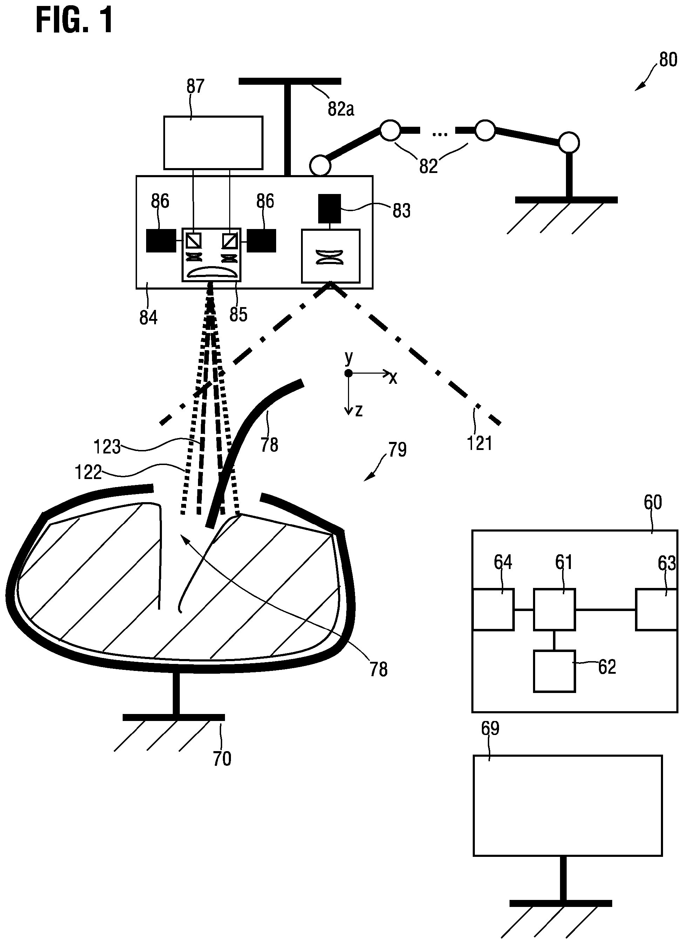

FIG. 1 illustriert schematisch Aspekte in Bezug auf ein beispielhaftes Operationsmikroskopiesystem 80. Das Operationsmikroskopiesystem 80 dient zur mikroskopischen Abbildung eines Untersuchungsbereichs bei einem chirurgischen Eingriff. Dazu wird ein Patient 79 auf einem Operationstisch 70 platziert. Gezeigt ist der Situs 78 mit einem Operationsinstrument 78.

Das Operationsmikroskopiesystem 80 beinhaltet ein robotisches Stativ 82, welches ein positionierbares Kopfteil 81 trägt. Das robotische Stativ 82 kann, je nach Variante, unterschiedliche Freiheitsgrade aufweisen. Es sind robotische Stative 82 bekannt, die sechs Freiheitsgrade für die Positionierung des Kopfteils 81 aufweisen, d.h. Translation entlang jeder der x-Achse, y-Achse und z-Achse und Rotation um jede der x-Achse, y-Achse und z-Achse. Das robotische Stativ 82 kann, wie in FIG. 1 gezeigt, einen Handgriff 82a aufweisen.

Während in FIG. 1 ein robotisches Stativ 82 diskutiert wird, wäre es allgemein auch möglich, dass das ein nur teilweise robotisches oder manuelles Stativ verwendet wird.

Der Kopfteil 81 umfasst ein Mikroskop 84 auf, welches Optikkomponenten 85, wie beispielsweise eine Beleuchtungsoptik, eine Objektivoptik, eine Zoomoptik, usw., aufweist. Außerdem umfasst das Mikroskop 84 im dargestellten Beispiel eine Mikroskopkamera 86 (hier eine Stereokamera mit zwei Kanälen; es wäre aber auch eine Mono-Optik denkbar), mit der Bilder des Untersuchungsbereichs erfasst werden können und zum Beispiel auf einem Bildschirm 69 wiedergegeben werden können.

Deshalb wird das Mikroskop 84 auch als digitales Mikroskop bezeichnet. Gezeigt ist auch ein Gesichtsfeld 123 der Mikroskopkamera 86.

Im Beispiel der FIG. 1 umfasst das Mikroskop 84 auch ein Okular 87 mit einem zugehörigen Gesichtsfeld 122. Das Okular 87 ist also optional. Beispielsweise kann der Detektionsstrahlengang mittels eines Strahlteilers geteilt werden, so dass sowohl ein Bild durch die Kamera 86 erfasst werden kann, wie auch eine Betrachtung durch das Okular 87 möglich ist. Es ist nicht in allen Varianten erforderlich, dass das Mikroskop 84 ein Okular aufweist. Es sind auch rein digitale Mikroskope 84 möglich, bei denen kein Okular vorhanden ist.

Im Beispiel der FIG. 1 umfasst das vom Stativ 82 getragene Kopfteil 81 des Operationsmikroskopiesystems 80 auch eine Umfeldkamera 83. Die Umfeldkamera 83 ist optional. Während die Umfeldkamera 83 im Beispiel der FIG. 1 in das Mikroskop 84 integriert dargestellt sind, wäre es möglich, dass diese getrennt vom Mikroskop 84 angeordnet ist. Die Umfeldkamera kann z.B. eine CCD-Kamera sein. Die Umfeldkamera kann auch eine Tiefenauflösung aufweisen. Alternativ oder zusätzlich zur Umfeldkamera wäre es auch denkbar, das andere assistierende Sensoren vorhanden sind, z.B. ein Abstandssensor (etwa eine Lichtlaufzeitkamera oder einen Ultraschallsensor oder einen Sensor mit strukturierter Beleuchtung). Ein Gesichtsfeld 121 der Umfeldkamera 83 ist in FIG. 1 gezeigt.

Der Chirurg hat somit mehrere Möglichkeiten, den Untersuchungsbereich zu betrachten: Über das Okular 87, über das Mikroskopbild, das von der Kamera 86 aufgenommen wird, oder über ein Übersichtsbild, das von einer Umgebungskamera 83 aufgenommen wird. Der Operateur kann den Untersuchungsbereich auch direkt (ohne Vergrößerung) betrachten.

Als nächstes werden Aspekte in Bezug auf die Gesichtsfelder 121, 122 und 123 diskutiert.

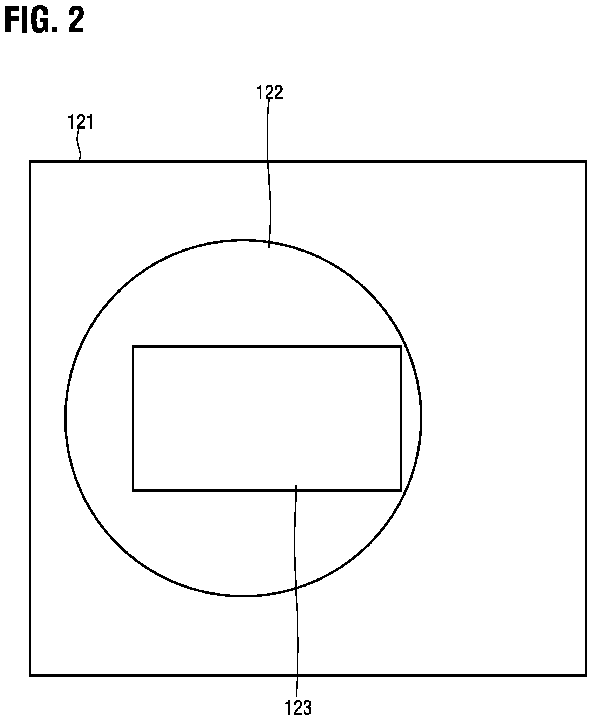

FIG. 2 illustriert Aspekte in Bezug auf die verschiedenen Gesichtsfelder. In FIG. 2 ist das Gesichtsfeld 121 einer Umfeldkamera des Operationsmikroskopiesystems 80 dargestellt. Übersichtsbilder bilden also einen relativ großen Bereich ab. Außerdem ist beispielhaft das Gesichtsfeld 122 des Okulars und das Gesichtsfeld 123 der

Mikroskopkamera 86 dargestellt. Die Gesichtsfelder 121, 122, 123 müssen nicht aneinander zentriert angeordnet sein.

Wieder Bezug nehmend auf FIG. 1: Die verschiedenen Komponenten des Operationsmikroskopiesystems 80 wie beispielsweise das robotische Stativ 82, das Mikroskop 84 oder die ein oder mehreren weiteren Komponenten wie z.B. die Umfeldkamera 83 werden von einem Prozessor 61 einer Datenverarbeitungseinrichtung 60 gesteuert.

Der Prozessor 61 kann beispielsweise als allgemeiner Zentralprozessor (CPU) und/oder als feldprogrammierbarer Logikbaustein (FPGA) und/oder als anwendungsspezifischer integrierter Schaltkreis (ASIC) ausgebildet sein. Der Prozessor 61 kann Programmcode aus einem Speicher 62 laden und ausführen.

Der Prozessor 61 kann mit verschiedenen Komponenten des Operationsmikroskopiesystems 80 über eine Kommunikationsschnittstelle 64 kommunizieren. Beispielsweise kann der Prozessor 61 das Stativ 82 ansteuern, um das Kopfteil 81 in Bezug auf den Operationstisch 70 zu bewegen, beispielsweise translatorisch und/oder rotatorisch. Der Prozessor 71 kann zum Beispiel Optikkomponenten 85 des Mikroskops 84 ansteuern, um einen Zoom und/oder Fokus (Brennweite) zu verändern. Es könnten Bilder der Umfeldkamera, sofern vorhanden, ausgelesen und ausgewertet werden. Allgemein können Bilder ausgewertet werden und basierend auf der Auswertung eine Assistenzfunktionalität ausgeführt werden.

Ferner umfasst die Datenverarbeitungseinrichtung 60 eine Benutzerschnittstelle 63. Über die Benutzerschnittstelle 63 können Befehle eines Chirurgen oder allgemein eines Benutzers des Operationsmikroskopsystems 80 empfangen werden. Die Benutzerschnittstelle 63 kann verschiedene Konfigurationen aufweisen. Beispielsweise kann die Benutzerschnittstelle 63 eine oder mehrere der folgenden Komponenten umfassen: Griffe am Kopfteil 81; Fußschalter; Spracheingabe; Eingabe über eine graphische Benutzeroberfläche; etc. Es wäre möglich, dass die Benutzerschnittstelle 63 eine graphische Interaktion über Menüs und Knöpfe auf dem Monitor 69 bereitstellt.

Nachfolgend werden Techniken beschrieben, wie es mittels des Operationsmikroskopiesystems 80 möglich ist, eine Assistenzfunktionalität vorzubereiten. Die Assistenzfunktionalität kann z.B. durch einen Benutzerbefehl angefordert werden.

Ein solcher Benutzerbefehl kann, als allgemeine Regel, unterschiedliche Ausprägungen aufweisen. Beispielsweise könnte der Benutzerbefehl ein bestimmtes Objekt spezifisch identifizieren. Der Benutzer könnte z.B. per Sprachbefehl angeben: "Auto-Zentrierung auf das Operationsinstrument 1". Ein solcher Benutzerbefehle gibt also genau an, in Bezug auf welches Objekt eine Konfiguration des Operationsmikroskopiesystems 80 zu erfolgen hat (hier auf das "Operationsinstrument 1"). In anderen Beispielen wäre es aber auch denkbar, dass der Benutzerbefehle unspezifisch für ein bestimmtes Objekt ist. Der Benutzer könnte zum Beispiel per Sprachbefehl oder per Knopfdruck angeben: "Auto-Zentrierung". Es wird also nicht angegeben, auf welches Objekt die Auto-Zentrierung erfolgen soll (auch wenn mehrere Objekte sichtbar sind, die Kandidaten für die Auto-Zentrierung sind). Der Benutzerbefehl ist in diesem Beispiel nicht eindeutig. Der Benutzerbefehl unterscheidet nicht zwischen mehreren sichtbaren Operationsinstrumenten. In Referenzimplementierungen kann dann der Benutzerbefehl falsch interpretiert werden und z.B. eine Auto-Zentrierung in Bezug auf das falsche Operationsinstrument vorgenommen werden. Die Benutzererwartung (z.B. Auto-Zentrierung auf "Operationsinstrument 1") entspricht dann unter Umständen nicht dem tatsächlichen Systemverhalten (z.B. Auto-Zentrierung auf den geometrischen Mittelpunkt aller sichtbaren Operationsinstrumente).

Nachfolgend werden Techniken beschrieben, die es im Zusammenhang mit einer durch einen Benutzerbefehl Assistenzfunktionalität ermöglichen, die Benutzererwartung besser als in Referenzimplementierungen zu erfüllen. Ein deterministischen Systemverhalten wird ermöglicht, welches in unterschiedlichsten Situationen und/oder in Anbetracht unterschiedlichster Szenen reproduzierbare und nachvollziehbare Ergebnisse liefert. Solche Techniken beruhen auf der Erkenntnis, dass es insbesondere in Hoch-Stress-Situationen unter Zeitdruck, wie sie typischerweise in einem chirurgischen Umfeld auftreten, notwendig ist, dass das Systemverhalten einer automatischen Steuerung genau mit der Benutzererwartung übereinstimmt.

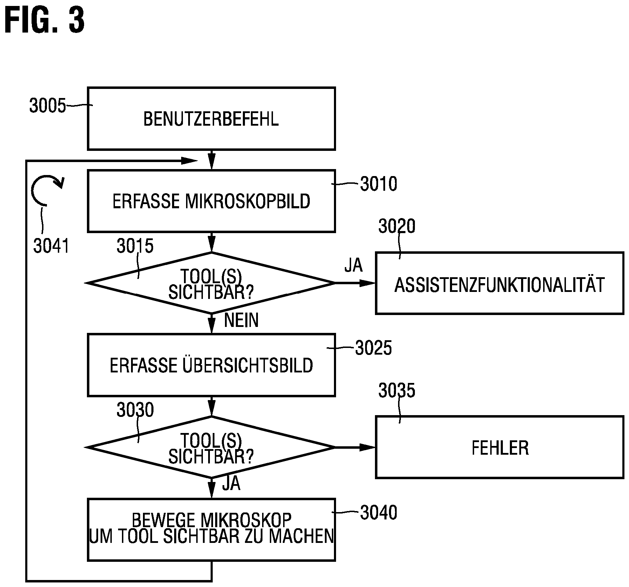

FIG. 3 ist ein Flussdiagramm eines beispielhaften Verfahrens. Das Verfahren der FIG. 3 betrifft Techniken im Zusammenhang mit der Konfiguration eines Operationsmikroskopiesystems zur Bildgebung eines Objekts in Erwiderung auf einen Benutzerbefehl.

Das Verfahren aus FIG. 3 kann von einem Prozessor einer Datenverarbeitungseinrichtung ausgeführt werden, beispielsweise vom Prozessor 61 der Datenverarbeitungseinrichtung 60 des Operationsmikroskopiesystems 80 aus dem Beispiel der FIG. 1. Der Prozessor kann dazu Programmcode aus einem Speicher laden und anschließend ausführen.

In Box 3005 wird ein Benutzerbefehl empfangen. Der Benutzerbefehl kann eine Assistenzfunktionalität anfordern. Der Benutzerbefehl fordert beispielsweise die Konfiguration der Mikroskopkamera zur Bildgebung eines Objekts, beispielsweise eines Operationsinstruments, implizit oder explizit an. Der Benutzerbefehl wird von einer Benutzerschnittstelle empfangen. Der Benutzerbefehl kann zum Beispiel eine Auto-Ausrichtung oder eine Auto-Fokussierung anfordern. Der Benutzerbefehl könnte die Vermessung eines Operationsinstruments anfordern.

Der Benutzerbefehl kann unspezifisch für ein bestimmtes Objekt sein. Der Benutzerbefehl kann nicht angeben, auf welches von mehreren sichtbaren Objekten er sich bezieht.

In Box 3010 (optionale Box) wird ein Mikroskopbild erfasst. Dazu wird die Mikroskopkamera des Mikroskops angesteuert, vergleiche Mikroskopkamera 87 des Mikroskops 84 im Beispiel der FIG. 1. Wie voranstehend bereits im Zusammenhang mit FIG. 2 diskutiert wurde, weist die Mikroskopkamera typischerweise ein vergleichsweise kleines Gesichtsfeld auf, nämlich insbesondere ein Gesichtsfeld, welches kleiner ist als das Gesichtsfeld eines Okulars oder einer Umfeldkamera (sofern vorhanden).

In Box 3015 (optionale Box) wird dann das durch den Benutzerbefehl identifizierte Objekt, beispielsweise ein Operationsinstrument, in dem Mikroskopbild aus Box 3010 gesucht. Wenn das Objekt bereits im Gesichtsfeld der Mikroskopkamera positioniert ist, wird das Objekt in Box 3015 gefunden, das heißt es ist im Mikroskopbild sichtbar: Dann wird Box 3020 ausgeführt. Dort wird eine Assistenzfunktionalität basierend auf dem Mikroskopbild ausgeführt (z.B. eine Auto-Zentrierung oder Auto-Fokussierung oder eine Vermessung des Objekts).

Es können Szenarien auftreten, bei denen in Box 3015 das Objekt im Mikroskopbild nicht gefunden wird. Das bedeutet, dass das Objekt nicht im zentralen Bereich der Szene positioniert ist, der von der Mikroskopkamera abgebildet wird. In einem solchen Fall wird in Box 3025 die Umfeldkamera zum Erfassen eines Übersichtsbilds angesteuert. Dies wird getan, um zu überprüfen, ob das Objekt im peripheren Bereich der Szene positioniert ist, der von der Umfeldkamera aber nicht von der Mikroskopkamera abgebildet wird.

Dann kann in Box 3030 bestimmt, ob Objekt im Übersichtsbild sichtbar ist. In Box 3030 kann bestimmt werden, ob sich das Objekt im peripheren Bereich befindet, das heißt in demjenigen Bereich der Szene, die vom Gesichtsfeld der Umfeldkamera abgedeckt wird, jedoch nicht vom Gesichtsfeld des Mikroskops (in FIG. 2 ist das derjenige Bereich, der außerhalb des Gesichtsfelds 123 aber innerhalb des Gesichtsfelds 121 liegt).

Das Objekt wird also, allgemein formuliert, im Übersichtsbild gesucht. Wenn das Objekt nicht im Übersichtsbild gefunden wird, wird in Box 3035 ein Fehler ausgegeben. Andernfalls wird Box 3040 ausgeführt.

In Box 3040 wird ein Steuerbefehl für das robotische Stativ zum Bewegen des Mikroskops bereitgestellt, so dass das Objekt im Gesichtsfeld der Mikroskopkamera positioniert ist, das heißt in einem zentralen Bereich der Szene. Das bedeutet, dass in Box 3040 eine Grob-Ausrichtung erfolgt, so dass anschließend das Operationsinstrument auch in einem Mikroskopbild, welches in einer weiteren Iteration 3041 von Box 3010 erfasst wird, sichtbar ist.

Zusammenfassend ist es mittels des Verfahrens in FIG. 3 also möglich, mittels der Umfeldkamera zunächst ein Übersichtsbild zur Lokalisierung von Operationsinstrumenten für assistierende Funktionen zu erfassen. In diesem Fall wird zunächst dieses Übersichtsbild der Umfeldkamera evaluiert und bestimmt, ob sich dort ein Operationsinstrument befindet. Daraufhin wird das robotische Stativ angesteuert, um das erkannte Operationsinstrument in das Gesichtsfeld der Mikroskopkamera zu bewegen. Dann kann die eigentliche Assistenzfunktionalität durchgeführt werden, basierend auf einer Auswertung von ein oder mehreren Mikroskopbildern.

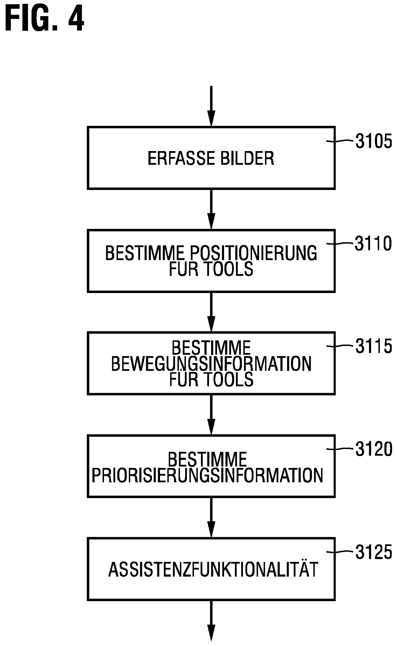

Manchmal kann es vorkommen, dass der Benutzerbefehl aus Box 3005 nicht genau das mindestens eine Objekt angibt, in Bezug auf welches die Assistenzfunktionalität in Box 3020 erfolgen soll. Beispielsweise könnten vier Operationsinstrumente sichtbar sein, die alle Kandidaten für die Assistenzfunktionalität sind (z.B. eine Auto-Zentrierung). Damit ist zunächst unklar, in Bezug auf welche Teilmenge der sichtbaren Operationsinstrumente ggf. in Box 3030 eine Suche und anschließend in Box 3040 eine Positionierung zu erfolgen hat. Insbesondere um solche Probleme zu lösen, können Techniken angewendet werden, wie sie nachfolgend im Zusammenhang mit FIG. 4 beschrieben werden.

FIG. 4 ist ein Flussdiagramm eines beispielhaften Verfahrens. Das Verfahren der FIG. 4 betrifft Techniken, um das Ausführen einer Assistenzfunktionalität vorzubereiten. Die Assistenzfunktionalität kann z.B. die die Position und/oder Orientierung (Positionierung; auch als Pose oder Lage bezeichnet) und/oder andere geometrische Eigenschaften von Operationsinstrumenten verwenden - auch dann, wenn eine Vielzahl von solchen Operationsinstrumenten in den entsprechenden Bildern, zum Beispiel Übersichtsbildern oder Mikroskopbildern, sichtbar sind. Alternativ oder zusätzlich wäre es denkbar, dass die Assistenzfunktionalität Bewegungsinformation berücksichtigt, die anhand einer Abfolge von Bildern bestimmt wird.

Die Techniken der FIG. 4 betreffen Aspekte, um gegebenenfalls Zweideutigkeiten aufgrund der Vielzahl von entsprechenden sichtbaren Operationsinstrumenten zu vermeiden.

Aspekte der FIG. 4 können z.B. im Zusammenhang mit der Lokalisierung von Operationsinstrumenten in Box 3030, 3040 aus FIG. 3 verwendet werden. Aspekte der FIG. 4 können alternativ oder zusätzlich im Zusammenhang mit der Assistenzfunktionalität in Box 3020 aus FIG. 3 verwendet werden. FIG. 4 kann aber auch eigenständig ausgeführt werden, d.h. nicht im Kontext der FIG. 3.

Die verschiedenen Varianten in FIG. 4 werden im Zusammenhang mit einer Implementierung von Objekten als Operationsinstrumente beschrieben.

Entsprechende Techniken könnten aber auch für andere Typen von Objekten durchgeführt werden.

Beispiele für Operationsinstrumente sind allgemein: Skalpell, Pinzette, Schere, Nadelhalter, Klemme, Sauger, Trokar, Coagulator, Elektrokauter, Retraktor, Bohrer, Spreizer, Osteotom, Nahtmaterial, Knotenschieber, Raspatorium, Hämostat, Lanzette, Drainage, Fadenschneider, Spatel, Ultraschallaspiratoren.

Das Verfahren der FIG. 4 kann von einem Prozessor einer Datenverarbeitungseinrichtung ausgeführt werden, beispielsweise vom Prozessor 61 der Datenverarbeitungseinrichtung 60 des Operationsmikroskopiesystems 80 aus dem Beispiel der FIG. 1. Der Prozessor kann dazu Programmcode aus einem Speicher laden und den Programmcode ausführen.

In Box 3105 wird eine Abfolge von Bildern erfasst. Dies kann z.B. durch einen Benutzerbefehl ausgelöst werden, z.B. wie im Zusammenhang mit Box 3005 aus FIG. 3 beschrieben.

Das Verfahren aus FIG. 4 kann zum Beispiel durch einen Benutzerbefehl ausgelöst werden, der eine bestimmte Assistenzfunktionalität anfordert. Beispielsweise könnte der Benutzer eine Auto-Ausrichtung des Gesichtsfelds der Mikroskopkamera und/oder einer Auto-Fokussierung anfordern. Als allgemeine Regel wäre es denkbar, dass der Benutzerbefehl das spezifische Objekt, welches der Assistenzfunktionalität zugrunde zu legen ist bzw. diese definiert, nicht angibt. Das bedeutet also, dass ein Benutzerbefehl empfangen werden kann, der die Assistenzfunktionalität in Bezug auf ein unbestimmtes der dargestellten Objekte anfordert. Der Benutzerbefehl kann also eine Zweideutigkeit in Bezug auf die zu berücksichtigenden Operationsinstrumente aufweisen.

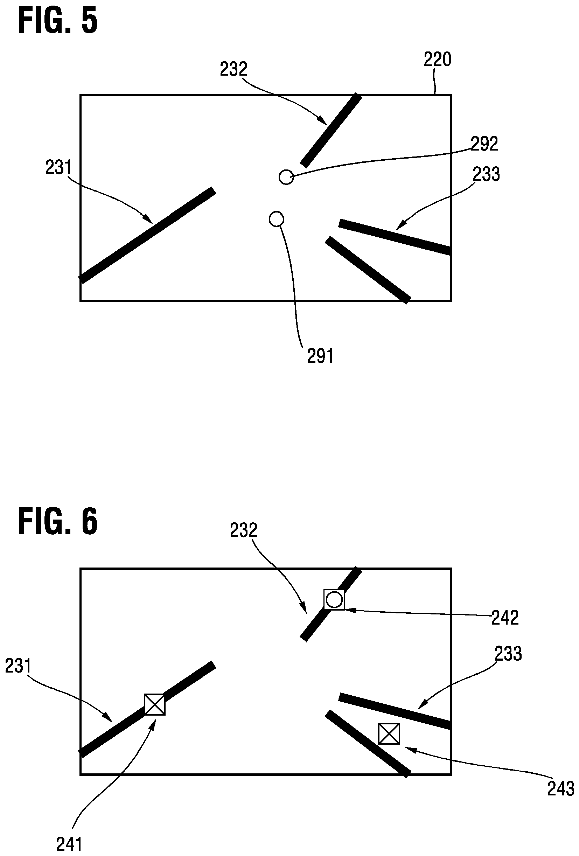

In Box 3105 wird eine Kamera, z.B. eine Mikroskopkamera oder eine Umfeldkamera, angesteuert. Die Bilder bilden eine chirurgische Szene über einen bestimmten Zeitraum ab. Ein beispielhaftes Bild 220 ist in FIG. 5 dargestellt. In FIG. 5 ist ersichtlich, dass insgesamt drei Operationsinstrumente 231, 232, 233 sichtbar sind.

Wieder Bezug nehmend auf FIG. 4: In der optionalen Box 3110 werden die sichtbaren Operationsinstrumente lokalisiert und optional deren Orientierung bestimmt. In Box 3110 kann also Positionierungsinformation bestimmt werden. Solche Positionierungsinformation kann eine Lokalisierung der Operationsinstrumente in den ein oder mehreren Bildern aus Box 3105 umfassen. Eine solche Positionierung kann eine Orientierung der Operationsinstrumente in den zwei oder mehr Bildern den Bildern aus Box 3105 umfassen. Die Positionierungsinformation kann zum Beispiel mittels einer Punktlokalisation oder mittels Bounding-Boxen erfolgen. Es könnte auch eine Segmentierung von Objekten von Operationsinstrumenten in den Bildern durchgeführt werden. Es könnte eine Instanzsegmentierung durchführt werden.

Die Positionierung kann z.B. basierend auf einem optischen Fluss ermittelt werden. Beispielsweise können z.B. Techniken eingesetzt werden, wie sie im Zusammenhang mit WO 2022/161930 A1 offenbart sind.

Die Positionierung kann optional auch in einem Referenzkoordinatensystem erfolgen. Beispielsweise kann basierend auf der Positionierung von Operationsinstrumenten in den Bildern aus Box 3105 bei Kenntnis der Pose der entsprechenden Kamera und der Bildgebungseigenschaften der Kamera auf eine Absolutpositionierung der Operationsinstrumente in einem Referenzkoordinatensystem zurückgeschlossen werden. Eine solche Technik kann insbesondere im Zusammenhang mit Bildern, die mittels einer Umfeldkamera erfasst werden, verwendet werden.

Dann wird in Box 3115 Bewegungsinformation für die Operationsinstrumente aus der Abfolge der Bilder bestimmt. Es wäre denkbar, aber nicht notwendig, dass die Bewegungsinformation basierend auf der Positionierungsinformation aus Box 3110 bestimmt wird. Wie obenstehend bereits erläutert ist Box 3110 optional und es ist möglich, dass die Positionierungsinformation nicht für das Bestimmen der Bewegungsinformation benötigt wird. Beispielsweise kann die Bewegungsinformation auch ohne eine vorherige Lokalisierung bestimmt werden, z.B. basierend auf dem optischen Fluss zwischen zwei nacheinander erfassten Bildern. Details zur Bestimmung der Bewegungsinformation werden später erläutert.

Dann wird in Box 3120 - basierend auf der Bewegungsinformation - Priorisierungsinformation bestimmt.

Dann wird basierend auf der Positionierung aus Box 3110 und/oder der Bewegungsinformation aus Box 3115, sowie basierend auf der Priorisierungsinformation aus Box 3120 eine Assistenzfunktionalität ausgeführt (in Box 3125). Beispielsweise können eine Auto-Ausrichtung und insbesondere eine Auto-Zentrierung auf das Aktivitätszentrum von Operationsinstrumenten erfolgen. Dazu können z.B. ein oder mehrere Aktivitätsbereiche aus der Bewegungsinformation bestimmt werden und deren geometrischer Mittelpunkt oder geometrischer Schwerpunkt als Aktivitätszentrum verwendet werden. Es könnte eine Auto-Ausrichtung und insbesondere eine Auto-Zentrierung auf ein bestimmtes Operationsinstrument oder ein geometrisches Zentrum mehrerer Operationsinstrumente erfolgen. Es könnte eine Auto-Fokussierung auf ein bestimmtes Operationsinstrument erfolgen, z.B. auf dessen Spitze.

Die Assistenzfunktionalität bezieht sich dabei auf die Operationsinstrumente, z.B. auf der Positionierungsinformation oder aber auch auf andere geometrische Eigenschaften der Operationsinstrumente (z.B. Abstandsmessung zwischen Operationsinstrumenten oder Öffnungswinkel eines Klammerinstruments, usw.)

Verschiedene Beispiele beruhen auf der Erkenntnis, dass es in bestimmten Varianten hilfreich sein kann, wenn die Assistenzfunktionalität nur eine Teilmenge aller im entsprechend Bild sichtbaren Operationsinstrumente berücksichtigt oder, allgemeiner formuliert, basierend auf der Priorisierungsinformation aus Box 3110 ausgeführt wird. Das bedeutet beispielsweise, dass die Positionierung und/oder Bewegungsinformation eines bestimmten Operationsinstruments der sichtbaren Operationsinstrumente stärker berücksichtigt wird als die Positionierung und/oder Bewegungsinformation eines anderen Operationsinstruments der sichtbaren Operationsinstrumente (dessen Positionierung und Bewegungsinformation ggf. auch überhaupt nicht berücksichtig wird).

Es kann also, in anderen Worten und allgemeiner formuliert, eine Unterscheidung zwischen relevanteren und weniger relevanten Operationsinstrumenten erfolgen; relevantere Operationsinstrumente werden dann bei der Assistenzfunktionalität stärker berücksichtigt als weniger relevanterer Operationsinstrumente.

Dies wird am Beispiel der FIG. 5 in einem konkreten Beispiel erläutert: In der beispielhaften Szene der FIG. 5 befinden sich ein Sauger 231 (Zweck: Blut absaugen), ein Bipolar-Coagulator 233 (Zweck: Blutung veröden) sowie ein Retraktor 232 (Zweck: Hirngewebe zurückhalten). Für den Chirurg sind die relevanten Instrumente der Sauger sowie der Bipolar-Coagulator, da diese im gezeigten Bild die primäre chirurgische Aktion durchführen (veröden, absaugen). Der Retraktor ist in dieser Szene jedoch nicht relevant: Der Retraktor hält das passiv Hirngewebe zurück, er ist räumlich fixiert und er führt keine primäre chirurgische Aktion durch. Das ist nur ein Beispiel für die Priorisierung relevanter Operationsinstrumente. Alternativ können auch Instrumente des assistierenden Chirurgen im Bild sichtbar sein, die jedoch für den Hauptchirurgen nur geringe Relevanz haben. Im Beispiel der FIG. 5 soll nun der geometrische Schwerpunkt der chirurgisch relevanten Instrumente bestimmt werden und anschließend eine Auto-Zentrierung auf diesen geometrischen Schwerpunkt durchgeführt werden. Der geometrische Schwerpunkt 291 nur der chirurgisch-relevanten Instrumente (d.h. ohne den Retraktor 232) ist beabstandet vom geometrischen Schwerpunkt 292 aller Instrumente 231, 232, 233. Aus Nutzersicht kann bei einer falschen oder unterbliebenen Priorisierung der Instrumente 231, 233 gegenüber dem Operationsinstrument 232 also eine schlechte Auto-Zentrierung erfolgen, weil anschließen der Schwerpunkt 292 zentriert ist, anstatt der Schwerpunkt 291. Ein entsprechendes Szenario lässt sich auch im Zusammenhang mit der Auto-Fokussierung beschreiben. Im Vergleich zu der voranstehend geschilderten Variante, bei der eine Auto-Zentrierung auf den geometrischen Schwerpunkt 291 erfolgt, kann es vorteilhaft sein, eine Auto-Fokussierung auf die Spitze oder eine andere charakteristische Position des einen höchst priorisierten Instruments durchzuführen (also zum Beispiel auf das Operationsinstrument 231). Dies beruht auf der Erkenntnis, dass der Chirurg typischerweise mit einem einzigen Instrument primär operiert und andere Instrumente sekundär hinter diesem primären Instrument zurücktreten.

Verschiedene Beispiele beruhen auf der Erkenntnis, dass es basierend auf der Bewegungsinformation besonders gut möglich ist, eine Unterscheidung zwischen relevanten und weniger relevanten Operationsinstrumenten zu treffen. In anderen Worten ist es basierend auf der Bewegungsinformation zuverlässig möglich, Priorisierungsinformation zu bestimmen.

Es sind verschiedene Implementierungen der Bewegungsinformation denkbar und einige Beispiele werden nachfolgend diskutiert. Diese Beispiele können auch miteinander kombiniert werden.

Beispielsweise kann die Bewegungsinformation einen optischen Fluss zwischen aufeinanderfolgenden Bildern der Abfolge von Bildern umfassen. Die Bewegungsinformation kann ein oder mehrere Aktivitätsbereich angeben. Entsprechende Techniken sind im Detail offenbart in WO2022161930 A1, deren Offenbarungsgehalt hierin durch Querverweis aufgenommen wird.

Beispielsweise wäre es denkbar, dass die Bewegungsinformation indikativ für eine zeitgemittelte Bewegungsmagnitude der Bewegung jedes einzelnen Objekts der Objekte ist. Eine solche Bewegungsmagnitude kann zum Beispiel derart bestimmt werden, dass mehrere Objekte lokalisiert werden (vgl. Box 3110) und dann jeweils innerhalb eines entsprechenden Bereichs, in dem das entsprechende Objekt lokalisiert ist, die Bewegung des Objekts nachverfolgt/getrackt wird. Allgemein formuliert kann also die Bewegungsinformation basierend auf einer Positionierung der Objekte bestimmt werden.

Die Bewegungsinformation kann z.B. indikativ für ein oder mehrere Bewegungsmuster der Objekte sein. Beispielsweise ist es denkbar, dass ein bestimmter Typ von chirurgischem Werkzeug - z. B. ein Sauger - bevorzugt in einer kreisförmigen Bewegung eingesetzt wird; ein solches Bewegungsmuster (kreisförmige Bewegung) kann dann erkannt und z. B. daraus geschlossen werden, dass der Sauger ein Hilfswerkzeug mit niedriger Priorität ist. Andererseits könnte ein anderer Typ von chirurgischem Werkzeug, z. B. ein Skalpell, primär translatorisch bewegt werden, d. h. hin und her bewegt werden. Ein solches Bewegungsmuster (translatorische Bewegung zwischen zwei Endpunkten) kann dann durch ein erkannt und z. B. darauf geschlossen werden, dass das Skalpell ein primäres Werkzeug mit hoher Priorität ist. Generell können wiederkehrende Bewegungsmuster, die durch den chirurgischen Einsatz eines bestimmten Werkzeugs vorgegeben sind, bei der Priorisierung eines Werkzeugs berücksichtigt werden. Solche Bewegungen des Werkzeugs dienen der Anwendung des Werkzeugs im chirurgischen Ablauf selbst (z. B. im obigen Beispiel des Saugers zum Absaugen von Blut oder im Zusammenhang mit dem obigen Beispiel des Skalpells zum Schneiden von Gewebe); sie sind also keine Bewegungen, die im Rahmen einer spezifischen Gestenerkennung ausgeführt werden und keinen über die Ausführung der Geste hinausgehenden Selbstzweck haben.

Weitere Beispiele für Bewegungsinformation wären zum Beispiel Information betreffend die Richtungsverteilung einer Bewegung des entsprechenden Objekts. Es wäre auch denkbar, anzugeben, welche Bewegungsfrequenzen die Bewegung des Objekts aufweist.

Voranstehend wurden verschiedene beispielhafte Implementierungen für die Bewegungsinformation offenbart. Auch Kombinationen aus solchen offenbarten Varianten für die Bewegungsinformation können in den verschiedenen hierin beschriebenen Techniken eingesetzt werden.

Es gibt verschiedene Möglichkeiten, um die Priorisierungsinformation zu implementieren. Beispielsweise könnte eine Segmentierungskarte ausgegeben werden, die in Bezug auf eines der erfassten Bilder die Bereiche des Bilds, in denen die Objekte angezeigt werden, in unterschiedliche Prioritätsklassen einteilt. Es wäre auch denkbar, dass Label ausgegeben werden, die an Objektpositionen angeordnet sind. Diese Label können dann die Prioritätsinformation anzeigen. Es wäre also denkbar, dass die Priorisierungsinformation eine Lokalisierung (z.B. Punktlokalisation, Bounding-Box, usw.) mit Priorisierungslabel umfasst. Ein entsprechendes Beispiel ist in FIG. 6 für eine Punktlokalisation dargestellt. Dort ist für die Operationsinstrumente 231, 232, 233 jeweils ein entsprechendes Label 241, 242, 243 an einer Mittelpunkt-Position der Operationsinstrumente 231, 232, 233 vorhanden. Diese Label 241-243 indizieren die Priorität der Operationsinstrumente, wobei die Label 241, 243 eine hohe Priorität indizieren; und das Label 242 eine niedrige Priorität indiziert. Die Priorisierungsinformation könnte zum Beispiel Aktivitätszentren für relevante und nicht-relevante Objekte anzeigen. Die Priorisierungsinformation kann also, allgemein formuliert, mit der Positionierung der verschiedenen Objekte verknüpft sein.

Die Priorisierungsinformation kann entweder einen kontinuierlichen Wert (zum Beispiel von 1 =niedrige Priorität; bis 10=hohe Priorität; Regression) annehmen kann oder auch eine Klassenzuweisung der Objekte in vorgegebene Klassen; z.B. wäre eine Klassenzuweisung in eine erste Klasse und eine zweite Klasse möglich (und optional ein oder mehrere weitere Klassen). Die Klassenzuweisung kann binär sein. Beispielsweise kann die erste Klasse relevante Objekte betreffen und die zweite Klasse nicht-relevante Objekte umfassen. Es ist dann möglich, dass die Assistenzfunktionalität ausschließlich basierend auf der Positionierung derjenigen ein oder mehreren Objekte erfolgt, die der ersten Klasse zugewiesen sind (das heißt solche Objekte, die der zweiten Klasse zugewiesen sind, werden ignoriert). Beispielsweise wurde im Zusammenhang mit FIG. 6 ein Szenario diskutiert, bei dem die Operationsinstrumente 231, 233 der ersten Klasse zugewiesen sind; und das Operationsinstrument232 der zweiten, nicht-relevanten Klasse zugewiesen ist.

Die Priorisierungsinformation kann auf unterschiedliche Weisen im Zusammenhang mit der Assistenzfunktionalität berücksichtigt werden. Nachfolgend werden einige Beispiele erläutert.

Die Assistenzfunktionalität kann z.B. die Vermessung eines Operationsinstruments betreffen. Dann kann z.B. dasjenige Operationsinstrument vermessen werden, welches die größte Priorität aufweist.

Die Assistenzfunktionalität kann auch eine automatische Konfiguration von ein oder mehreren Komponenten des Operationsmikroskopiesystems umfassen. Im Rahmen der Assistenzfunktionalität kann dann eine Soll-Konfiguration für ein oder mehrere Komponenten des Operationsmikroskopiesystems bestimmt werden. Bei der Bestimmung der Soll-Konfiguration kann die Positionierung solcher der zwei oder mehr Objekte stärker berücksichtigt werden, die basierend auf der Priorisierungsinformation höher priorisiert sind, im Vergleich zur Positionierung solcher der zwei oder mehr Objekte, die basierend auf der Priorisierungsinformation niedriger priorisiert sind. Es wäre ein gleitender Übergang zwischen stärkerer und schwächerer Berücksichtigung denkbar oder ein binärer Übergang, das heißt die Positionierung höher priorisierter Objekte wird berücksichtigt und die Positionierung niedriger priorisierter Objekte wird nicht berücksichtigt. Je nach Assistenzfunktionalität werden unterschiedliche Soll-Konfigurationen bestimmt. Beispielsweise könnte die Soll-Konfiguration des Operationsmikroskopiesystems eine Ausrichtung eines Gesichtsfelds des Mikroskops des Operationsmikroskopiesystems (zum Beispiel einer Mikroskopkamera oder eines Okulars) in Bezug auf mindestens eines der zwei oder mehr Operationsinstrumente, welches in Abhängigkeit von der Priorisierungsinformation ausgewählt ist, umfassen. Das bedeutet also, dass zum Beispiel der Schwerpunkt aller höher priorisierten Operationsinstrumente basierend auf den entsprechenden Positionen bestimmt wird und dann das robotische Stativ so angesteuert wird, dass der Schwerpunkt im Zentrum des Gesichtsfelds des Mikroskops liegt, wie obenstehend bereits im Zusammenhang mit FIG. 5 erläutert. Dies ist aber nur ein Beispiel. Es wäre auch denkbar, dass lediglich ein einziges Operationsinstrument aus der Vielzahl der sichtbaren Operationsinstrumente ausgewählt wird - nämlich dasjenige, mit der höchsten Priorisierung - und eine Auto-Anordnung des Gesichtsfelds des Mikroskops auf einen relevanten Punkt dieses ausgewählten Operationsinstruments erfolgt, beispielsweise auf dessen Spitze. In einer weiteren Variante umfasst die Soll-Konfiguration des Operationsmikroskopiesystems eine Auto-Fokussierung unter Berücksichtigung von mehreren Operationsinstrumenten, wobei innerhalb der Operationsinstrumenten eine Priorisierung erfolgt. So wäre es in einer weiteren Variante möglich, dass die Soll-Konfiguration des Operationsmikroskopiesystems eine Fokussierung des Mikroskops in Bezug auf eines der zwei oder mehr vorgegebenen Operationsinstrumente, das in Abhängigkeit der Priorisierungsinformation ausgewählt ist, umfasst. Es kann also ein Auto-Fokus in Bezug auf ein hochpriorisiertes Operationsinstrument bereitgestellt werden. Wird auf einen geometrischen Schwerpunkt von zwei oder mehr Operationsinstrumenten (oder allgemein Objekten) fokussiert, so kann zusätzlich zum geometrischen Schwerpunkt auch der Tiefenschwerpunkt für die Auto-Fokussierung berücksichtigt werden. Dies bedeutet also, dass für alle Objekte, die in die Bestimmung des örtlichen Schwerpunkts einfließen, die Tiefe bestimmt wird; und dann auf den Mittelwert fokussiert wird. Dies ist abzugrenzen von einer (grundsätzlich auch möglichen Variante), bei der auf die Tiefe am örtlichen Schwerpunkt fokussiert wird.

Als nächstes werden Details offenbart, wie das Mapping von Bewegungsinformation zu Priorisierungsinformation aussehen kann. D.h. es wird nachfolgend beschrieben, wie genau in Box 3120 die Priorisierungsinformation basierend auf der Bewegungsinformation bestimmt werden kann.

Im ersten Beispiel wird die Priorisierungsinformation basierend auf der Bewegungsinformation unter Verwendung eines vordefinierten Kriteriums bestimmt. Das bedeutet also in anderen Worten, dass das Kriterium, welches zur Bestimmung der Priorisierungsinformation anhand der Bewegungsinformation verwendet wird, unabhängig von der Bewegungsinformation selbst ist. Beispielsweise könnte ein fester Schwellenwert verwendet werden, welcher mit einem Wert der Bewegungsinformation verglichen wird. Es könnte eine Nachschlagetabelle verwendet werden, die unterschiedliche Werte der Bewegungsinformation unterschiedlicher Priorisierungsinformation zuordnet. Es könnte eine feste, vordefinierte Funktion verwendet werden, die Bewegungsinformation in Priorisierungsinformation übersetzt. Als Beispiel könnte zum Beispiel die Bewegungsmagnitude (zum Beispiel durch den optischen Fluss quantifiziert) berücksichtigt werden. Diese Bewegungsmagnitude könnte mit einem vorgegebenen Schwellenwert verglichen werden. Wenn die Bewegungsmagnitude größer ist als der vorgegebene Schwellenwert, so wird das entsprechende Operationsinstrument einer ersten, relevanten Klasse zugeordnet; ansonsten einer zweiten, nicht-relevanten Klasse. Bezugnehmend auf das Beispiel der FIG. 5: dort indiziert die Bewegungsmagnitude, dass der Sauger 231 und der Bipolar-Koagulator 233 signifikant bewegt werden (das heißt die Bewegungsmagnitude ist größer als ein Schwellenwert); der Retraktor 232 aber nicht. Der Retraktor 232 ist nämlich am Patienten fixiert und bewegt sich lediglich leicht mit dem Hirngewebe mit. In diesem Fall werden die Operationsinstrumente 231, 233 in der Klasse mit hoher Priorität und das Operationsinstrument232 in einer Klasse mit niedriger Priorität klassifiziert. Allgemein können feste Bewegungsschwellenwert verwendet werden, auch für anders definierte Bewegungsinformation. Ein solches Kriterium, welches in Bezug auf die Bewegungsinformation vordefiniert ist, kann von einem Benutzer eingestellt werden. Beispielverse können unterschiedliche Benutzer unterschiedliche Präferenzen in Bezug auf die Klassifikation von Operationsinstrumenten als relevant und nicht-relevant (das heißt in Bezug auf die Klassifikation von Operationsinstrumenten als mit hoher Priorität oder mit niedriger Priorität assoziiert) haben. Es wäre aber auch denkbar, dass ein solches Kriterium fest vorprogrammiert ist und nicht vom Benutzer verändert wird.

Im zweiten Beispiel (alternativ oder zusätzlich zum obigen ersten Beispiel) wird die Priorisierungsinformation basierend auf der Bewegungsinformation unter Verwendung von einem relativen Kriterium bestimmt. Das relative Kriterium wird basierend auf der Bewegungsinformation ermittelt. Das bedeutet in anderen Worten, dass z.B. ein relatives Verhältnis von Werten der Bewegungsinformation, die für verschiedene Operationsinstrumente ermittelt wird, berücksichtigt wird; oder ein Schwellenwert, der basierend auf den Werten der Bewegungsinformation angepasst wird (z.B. bei 50% des Maximums etc.). Beispielsweise kann eine Rangfolge von Werten der Bewegungsinformation berücksichtigt werden. Beispielsweise könnte lediglich ein einziges Objekt in eine erste Klasse, die mit hoher Priorität assoziiert ist, aufgenommen werden; ein solches Szenario bietet sich insbesondere für eine Auto-Fokussierung an. Es kann die Bewegung der verschiedenen Operationsinstrumente relativ zueinander berücksichtigt werden. Es kann berücksichtigt werden, ob sich die Operationsinstrumente aufeinander zu oder voneinander wegbewegen. In einer Variante wird das sich am schnellsten bewegende Objekt als relevant klassifiziert; alle übrigen Objekte werden als nicht-relevant klassifiziert; das bedeutet in anderen Worten, dass sich das am schnellsten bewegende Objekt in eine erste Klasse eingeordnet wird; und sämtliche andere Operationsinstrumente in eine zweite Klasse eingruppiert werden. Optional könnte auch ein Toleranzbereich verwendet werden (zum Beispiel 5 %). Sollte das sich am zweitschnellsten bewegende Objekt ähnlich schnell (zum Beispiel 95 % der Bewegungsamplitude des schnellsten Objekts) bewegen, wird der Schwerpunkt genommen. Sollte aber ein Objekt eine deutlich größere Bewegungsgeschwindigkeit aufweisen, als alle übrigen Objekte, wird lediglich dieses eine Objekt als relevant mit einer hohen Priorität klassifiziert. Es könnte ein Vergleich zwischen den Spektren der Bewegungsfrequenzen der verschiedenen Operationsinstrumente durchgeführt werden. Beispielsweise könnte überprüft werden, ob bestimmte Operationsinstrumente alle dasselbe Bewegungsspektrum aufweisen (was ein Indikator dafür wäre, dass diese Operationsinstrumente nicht vom Chirurgen geführt werden, sondern am Patienten fixiert sind und sich mit der Patientenbewegung mitbewegen).

Jetzt folgt ein praktisches Beispiel: Es könnte also zum Beispiel wiederum für das in FIG. 5 illustriert Szenario die Bewegungsmagnitude (zum Beispiel anhand des optischen Flusses) bestimmt werden. Die Rangfolge der Bewegungsmagnitude ist Sauger 231 - Bipolar-Coagulator 233 - Retraktor 232. Der Retraktor 232 weist also eine signifikant geringere Bewegungsmagnitude (relativ definiert) als der Sauger 231 und der Bipolar-Coagulator 233 auf. Deshalb ist dann denkbar, dass der Sauger 231 und der Bipolar-Coagulator, 233 einer Klasse mit hoher Priorität zugewiesen werden, der Retraktor 232 aber einer Klasse mit niedriger Priorität. In einem solchen Beispiel ist es entbehrlich, feste Schwellenwerte zu verwenden. Dies kann insbesondere dann hilfreich sein, wenn eine breite Vielzahl von unterschiedlichen Szenen berücksichtigt werden soll und es a-priori unbekannt ist, wie sich die Bewegungsmagnitude in Bezug auf die Relevanz der Objekte verhält. In einem solchen Fall ist es hilfreich, wenn, wie obenstehend beschrieben, ein relatives Kriterium verwendet wird, welches flexibel adaptiv auf alle Szenen anwendbar ist.

In einem dritten Beispiel (wiederum alternativ oder zusätzlich zu den oben diskutieren Beispielen) wird ein maschinengelerntes Modell verwendet werden, um die Priorisierungsinformation zu bestimmen. Das maschinengelernte Modell erhält als Eingabe die Bewegungsinformation. Das maschinengelernte Modell kann zum Beispiel trainiert sein, um Bewegungsmuster von relevanten Operationsinstrumenten zu erkennen und von Bewegungsmuster von weniger relevanten Operationsinstrumenten zu unterscheiden. Das maschinengelernte Modell kann dann die Priorisierungsinformation mit einer entsprechenden Klassenzuteilung ausgeben.

Ein maschinengelerntes Modell ermöglicht es, komplexere Entscheidungsregeln abzuleiten als in den voranstehend beschriebenen ersten und zweiten Beispielen. Beispielsweise können kumulativ ein oder mehrere der folgenden Faktoren berücksichtigt werden: Abstände der Instrumente bei der Bewegung; Bewegungsarten (zum Beispiel langsam versus schnell), Bewegungsrichtungen, Bildeinfallswinkel der Instrumente (um zwischen Assistent und Hauptchirurg zu differenzieren), Bewegungsmuster, usw. Die Berücksichtigung solche Kriterien kann durch ein geeignetes Training angelernt werden. Dazu kann ein Experte entsprechende Eingabedaten in das maschinengelernte Modell manuell annotiert, indem entsprechende Grundwahrheiten für die Priorisierungsinformation bestimmt werden.

Als allgemeine Regel kann das maschinengelernte Modell zusätzlich zur Eingabe der Bewegungsinformation noch weitere Eingaben erhalten. Beispiele wären zum Beispiel die Positionierung der zwei oder mehreren Operationsinstrumente , d.h. entsprechende Positionierungsinformation. Beispielsweise könnte eine entsprechende Bounding-Box oder eine Mittelpunktlokalisierung übergeben werden. Es könnte eine entsprechende Instanz-Segmentierungskarte übergeben werden.

Es könnte auch Information zu einem semantischen Kontext übergeben werden, also z.B. eine Operationsphase oder ein Operationstyp.

Voranstehend wurden Aspekte beschrieben, wie basierend auf der Bewegungsinformation die Priorisierungsinformation bestimmt wird. Im Zusammenhang mit den maschinengelernten Modellen wurde voranstehend bereits angedeutet, dass neben der Bewegungsinformation auch noch weitere Daten beim Bestimmen der Priorisierungsinformation berücksichtigt werden können (das gilt allgemein für die verschiedenen Beispiele und ist nicht beschränkt auf die Verwendung eines maschinengelernten Modells).

Beispielsweise kann die Priorisierungsinformation zusätzlich basierend auf der Positionierung der zwei oder mehreren Operationsinstrumente bestimmt werden. So wäre es denkbar, dass solche Objekte, die relativ zentral im Gesichtsfeld positioniert sind, tendenziell eine höhere Priorität aufweisen als peripher positionierte Objekte.

Es wäre alternativ oder zusätzlich auch möglich, die Priorisierungsinformation basierend auf einer semantischen Kontextinformation für eine mit den zwei oder mehr Operationsinstrumenten assoziierten Szene bestimmt wird. Beispiele für semantische Kontextinformation sind ein Typ der chirurgischen Intervention oder eine Information, die indikativ für die Phase der chirurgischen Intervention ist, zum Beispiel: "Koagulieren" oder "Blut absaugen" usw.). Es wäre auch denkbar, den OP-Typ zu identifizieren, also z.B. "spinal, cranio, tumor, vascular". Beispielsweise könnte entsprechende Kontextinformation als weitere Eingabe an ein maschinengelerntes Modell übergeben werden. Je nach semantischer Kontextinformation könnten zum Beispiel unterschiedliche Nachschlagetabellen für die Zuordnung von Bewegungsinformation zur Priorisierungsinformation verwendet werden. Je nach semantischer Kontextinformation könnten unterschiedliche Schwellenwerte für die Klassifizierung in eine höher priorisierte Klasse oder eine nieder priorisierte Klasse basierend auf einer Bewegungsmagnitude verwendet werden, um nur einige Beispiele zu nennen.

Als weiteres Beispiel könnte berücksichtigt werden, ob ein bestimmtes Operationsinstrument in der linken Hand oder in der rechten Hand geführt wird. Dies kann verglichen werden, mit einer entsprechenden Priorisierung der Hände des entsprechenden Chirurgen (das heißt ob der entsprechende Chirurg Linkshänder oder Rechtshänder ist).

Zusammenfassend erfolgt im Beispiel der FIG. 4, FIG. 5 und FIG. 6 die Bestimmung der Priorisierungsinformation basierend auf der Bewegungsinformation. Dies hat den Vorteil, dass eine besonders relevante und robuste Priorisierung erfolgen kann, die mit unterschiedlichen Typen von Objekten umgehen kann. Dies wird nachfolgend im Detail erläutert. Beispielsweise erfolgt in der US 10,769,443 B eine Unterscheidung zwischen "non-dominant" und "dominant" Instrumenten. Diese Unterscheidung erfolgt basierend auf drei Klassifikationen, nämlich erstens der Klassifikation des Werkzeugtyps, zweitens der Klassifikation, ob das Werkzeug assistiv ist oder nichtassistiv und drittens der Klassifikation, in welcher Hand (rechts / links) das Instrumente gehalten wird. Für die Entscheidung, ob ein Instrument "dominant" oder "non-dominant" ist bzw. "assistiv" oder "non-assistiv" ist, wird in der US 10,769,443 B also eine explizite Klassifikation des Instrumententyps (Sauger, Retraktor, ...) herangezogen. Der Typ des Instruments muss klassifiziert werden. In der Neurochirurgie gibt es jedoch >100 Typen von Instrumenten, welche visuell oft schwer zu unterscheiden sind. Eine explizite Klassifikation ist also technisch schwierig umsetzbar. Selbst wenn eine robuste explizite Klassifikation des Instrumententyps vorliegt, besteht ein weiteres Problem der Lösung in der US 10,769,443 B: Ein und dasselbe Instrument kann in manchen Situationen chirurgisch-relevant oder nicht-relevant sein. Beispielsweise ist ein Sauger dann relevant, wenn er gerade Blut absaugt; andererseits ist er nicht-relevant, wenn der Sauger dazu verwendet wird, lediglich Hirngewebe zurückzuhalten (wie eine Art handgehaltener, "dynamischer Retraktor") statt Blut abzusaugen. Des veranschaulicht, dass der Instrumententyp keine direkte Aussage über die chirurgische Relevanz des Instruments bzw. dessen Priorität bei der Assistenzfunktionalität erlaubt. Dieses Problem wird in der vorliegenden Offenbarung gelöst, indem die Priorisierungsinformation in Box 3120 basierend auf der Bewegungsinformation aus Box 3115 bestimmt wird.

Zusammenfassend wurden insbesondere die folgenden Beispiele beschrieben:
BEISPIEL 1. Computer-implementiertes Verfahren zum Steuern eines Operationsmikroskopiesystems (80) mit einem Stativ (82) und einem vom Stativ (82) getragenem Mikroskop (84),
   wobei das Verfahren umfasst:
   - Ansteuern (3105) einer Kamera (83, 86) des Operationsmikroskopiesystems zum Erhalten einer Abfolge von Bildern,
   - Bestimmen (3115) von Bewegungsinformation für jedes von zwei oder mehr in der Abfolge der Bilder abgebildeten Objekten (78, 231, 232, 233) basierend auf der Abfolge von Bildern,
   - Bestimmen (3120) von Priorisierungsinformation für die zwei oder mehr Objekte (78, 231, 232, 233) basierend auf der Bewegungsinformation, und
   - basierend auf der Priorisierungsinformation, Ausführen (3125) einer Assistenzfunktionalität im Zusammenhang mit den zwei oder mehr Objekten (78, 231, 232, 233).
BEISPIEL 2. Computer-implementiertes Verfahren nach BEISPIEL 1,
   wobei die Priorisierungsinformation eine Klassenzuweisung der zwei oder mehr Objekte (231, 232, 232) zumindest in eine erste Klasse und eine zweite Klasse umfasst,
   wobei die Assistenzfunktionalität im Zusammenhang mit mindestens einem Objekt (231, 233) ausgeführt wird, welches der ersten Klasse zugewiesen ist,
   wobei die Assistenzfunktionalität nicht im Zusammenhang mit mindestens einem weiteren Objekt (232) ausgeführt wird, welches der zweiten Klasse zugewiesen ist.
BEISPIEL 3. Computer-implementiertes Verfahren nach BEISPIEL 1 oder 2,
   wobei die Priorisierungsinformation basierend auf der Bewegungsinformation unter Verwendung von mindestens einem vordefinierten Kriterium bestimmt wird,
   wobei das mindestens eine vordefinierte Kriterium einen festen Bewegungsschwellenwert umfasst.
BEISPIEL 4. Computer-implementiertes Verfahren nach einem der voranstehenden BEISPIELE,
   wobei die Priorisierungsinformation ohne eine Klassifikation und insbesondere ohne Instanzsegmentierung der Objekte erfolgt.
BEISPIEL 5. Computer-implementiertes Verfahren nach einem der voranstehenden BEISPIELE,
   wobei die Priorisierungsinformation basierend auf der Bewegungsinformation unter Verwendung mindestens einem relativen Kriterium bestimmt, welches basierend auf der Bewegungsinformation bestimmt wird.
BEISPIEL 6. Computer-implementiertes Verfahren nach BEISPIEL 5,
   wobei das mindestens eine relative Kriterium eine Rangfolge der Bewegungsinformation der zwei oder mehr Objekte (231, 232, 233) umfasst.
BEISPIEL 7. Computer-implementiertes Verfahren nach BEISPIEL 5 oder 6,
   wobei das mindestens eine relative Kriterium eine relativ Bewegung der zwei oder mehr Objekte (231, 232, 233) zueinander umfasst.
BEISPIEL 8. Computer-implementiertes Verfahren nach einem der voranstehenden BEISPIELE,
   wobei die Priorisierungsinformation mittels eines maschinengelernten Modells bestimmt wird, welches als Eingabe die Bewegungsinformation erhält.
BEISPIEL 9. Computer-implementiertes Verfahren nach einem der voranstehenden BEISPIELE,
   wobei die Priorisierungsinformation weiterhin basierend auf einer Positionierung der zwei oder mehr Objekte (231, 232, 233) bestimmt wird.
BEISPIEL 10. Computer-implementiertes Verfahren nach einem der voranstehenden BEISPIELE,
   wobei die Priorisierungsinformation weiterhin basierend auf semantischer Kontextinformation für eine mit den zwei oder mehr Objekten assoziierte Szene bestimmt wird.
BEISPIEL 11. Computer-implementiertes Verfahren nach einem der voranstehenden BEISPIELE, wobei das Verfahren weiterhin umfasst:
   - Empfangen (3005) eines Benutzerbefehls, der die Assistenzfunktionalität in Bezug auf ein unbestimmtes der zwei oder mehr Objekten (231, 232, 233) anfordert.
BEISPIEL 12. Computer-implementiertes Verfahren nach einem der voranstehenden BEISPIELE,
   wobei die Bewegungsinformation einen optischen Fluss zwischen aufeinanderfolgenden Bildern der Abfolge von Bildern umfasst.
BEISPIEL 13. Computer-implementiertes Verfahren nach einem der voranstehenden BEISPIELE,
   wobei die Priorisierungsinformation mit einer Positionierung der zwei oder mehreren Objekten verknüpft ist.
BEISPIEL 14. Computer-implementiertes Verfahren nach einem der voranstehenden BEISPIELE,
   wobei die Priorisierungsinformation eine Segmentierungskarte mit Priorisierungslabel umfasst.
BEISPIEL 15. Computer-implementiertes Verfahren nach einem der voranstehenden BEISPIELE,
   wobei die Priorisierungsinformation eine Punktlokalisation mit Priorisierungslabel umfasst.
BEISPIEL 16. Computer-implementiertes Verfahren nach einem der voranstehenden BEISPIELE, wobei das Ausführen der Assistenzfunktionalität umfasst:
   - Bestimmen einer Soll-Konfiguration des Operationsmikroskopiesystems (80) basierend auf der Priorisierungsinformation und einer Positionierung der zwei oder mehr Objekten (231, 232, 233), und

   - Ansteuern von mindestens einer Komponente des Operationsmikroskopiesystems basierend auf der Soll-Konfiguration.
BEISPIEL 17. Computer-implementiertes Verfahren BEISPIEL 16, wobei die Soll-Konfiguration des Operationsmikroskopiesystems eine Ausrichtung eines Gesichtsfelds (122, 123) des Mikroskops (84) in Bezug auf mindestens eines der zwei oder mehr Objekten (231, 232, 233), das in Abhängigkeit von der Priorisierungsinformation ausgewählt ist, umfasst.
BEISPIEL 18. Computer-implementiertes Verfahren nach BEISPIEL 16 oder 17,
   wobei die Soll-Konfiguration des Operationsmikroskopiesystems eine Auto-Fokussierung des Mikroskops (84) in Bezug auf eines der zwei oder mehr Objekten (231, 232, 233), das in Abhängigkeit von der Priorisierungsinformation ausgewählt ist, umfasst.
BEISPIEL 19. Datenverarbeitungseinrichtung (60) zur Steuerung eines Operationsmikroskopiesystems (80), wobei die Datenverarbeitungseinrichtung (60) einen Prozessor (61) umfasst, der eingerichtet ist, um Programmcode aus einem Speicher (62) laden und ausführen kann, wobei das Ausführen des Programmcodes bewirkt, dass der Prozessor (61) die folgenden Schritte ausführt:
   - Ansteuern (3105) einer Kamera (83, 86) des Operationsmikroskopiesystems zum Erhalten einer Abfolge von Bildern,
   - Bestimmen (3115) von Bewegungsinformation für jedes von zwei oder mehr in der Abfolge der Bilder abgebildeten Objekten (78, 231, 232, 233) basierend auf der Abfolge von Bildern,
   - Bestimmen (3120) von Priorisierungsinformation für die zwei oder mehr Objekte (78, 231, 232, 233) basierend auf der Bewegungsinformation, und
   - basierend auf der Priorisierungsinformation, Ausführen (3125) einer Assistenzfunktionalität im Zusammenhang mit den zwei oder mehr Objekten (78, 231, 232, 233).
BEISPIEL 20. Datenverarbeitungseinrichtung (60) nach BEISPIEL 19, wobei das Ausführen des Programmcodes bewirkt, dass der Prozessor das Verfahren nach einem der BEISPIELE 1 bis 18 ausführt.
BEISPIEL 21. Operationsmikroskopiesystem, welches die Datenverarbeitungseinrichtung (60) nach BEISPIEL 19 oder 20 umfasst.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

Beispielsweise wurden voranstehend verschiedene Aspekte im Zusammenhang mit einer Assistenzfunktionalität beschrieben, die ein Operationsinstrument betrifft. Als allgemeine Regel wäre es aber denkbar, dass andere Typen von Objekte zu berücksichtigen, beispielsweise charakteristische anatomische Merkmale des Patienten.

Ferner wurden obenstehend verschiedene Aspekte im Zusammenhang mit einem robotischen Stativ beschrieben. Es ist nicht unbedingt notwendig, dass das Operationsmikroskopiesystem ein robotisches Stativ aufweist. Das Operationsmikroskopiesystem könnte auch ein teilweise robotisches Stativ oder ein manuelles Stativ aufweisen.

## Patentansprüche

1. Computer-implementiertes Verfahren zum Steuern eines Operationsmikroskopiesystems (80) mit einem Stativ (82) und einem vom Stativ (82) getragenem Mikroskop (84),
wobei das Verfahren umfasst:
- Ansteuern (3105) einer Kamera (83, 86) des Operationsmikroskopiesystems zum Erhalten einer Abfolge von Bildern,
- Bestimmen (3115) von Bewegungsinformation für jedes von zwei oder mehr in der Abfolge der Bilder abgebildeten Objekten (78, 231, 232, 233) basierend auf der Abfolge von Bildern,
- Bestimmen (3120) von Priorisierungsinformation für die zwei oder mehr Objekte (78, 231, 232, 233) basierend auf der Bewegungsinformation, und
- basierend auf der Priorisierungsinformation, Ausführen (3125) einer Assistenzfunktionalität im Zusammenhang mit den zwei oder mehr Objekten (78, 231, 232, 233).

2. Computer-implementiertes Verfahren nach Anspruch 1,
wobei die Priorisierungsinformation eine Klassenzuweisung der zwei oder mehr Objekte (231, 232, 232) zumindest in eine erste Klasse und eine zweite Klasse umfasst,
wobei die Assistenzfunktionalität im Zusammenhang mit mindestens einem Objekt (231, 233) ausgeführt wird, welches der ersten Klasse zugewiesen ist,
wobei die Assistenzfunktionalität nicht im Zusammenhang mit mindestens einem weiteren Objekt (232) ausgeführt wird, welches der zweiten Klasse zugewiesen ist.

3. Computer-implementiertes Verfahren nach Anspruch 1 oder 2,
wobei die Priorisierungsinformation basierend auf der Bewegungsinformation unter Verwendung von mindestens einem vordefinierten Kriterium bestimmt wird,
wobei das mindestens eine vordefinierte Kriterium einen festen Bewegungsschwellenwert umfasst.

4. Computer-implementiertes Verfahren nach einem der voranstehenden Ansprüche,
wobei die Priorisierungsinformation ohne eine Klassifikation und insbesondere ohne Instanzsegmentierung der Objekte erfolgt.

5. Computer-implementiertes Verfahren nach einem der voranstehenden Ansprüche,
wobei die Priorisierungsinformation basierend auf der Bewegungsinformation unter Verwendung mindestens einem relativen Kriterium bestimmt, welches basierend auf der Bewegungsinformation bestimmt wird,
wobei das mindestens eine relative Kriterium beispielsweise eine Rangfolge der Bewegungsinformation der zwei oder mehr Objekte (231, 232, 233) und/oder eine relativ Bewegung der zwei oder mehr Objekte (231, 232, 233) zueinander umfasst.

6. Computer-implementiertes Verfahren nach einem der voranstehenden Ansprüche,
wobei die Priorisierungsinformation mittels eines maschinengelernten Modells bestimmt wird, welches als Eingabe die Bewegungsinformation erhält.

7. Computer-implementiertes Verfahren nach einem der voranstehenden Ansprüche,
wobei die Priorisierungsinformation weiterhin basierend auf zumindest einem einer Positionierung der zwei oder mehr Objekte (231, 232, 233) und/oder basierend auf semantischer Kontextinformation für eine mit den zwei oder mehr Objekten assoziierte Szene bestimmt wird.

8. Computer-implementiertes Verfahren nach einem der voranstehenden Ansprüche, wobei das Verfahren weiterhin umfasst:
- Empfangen (3005) eines Benutzerbefehls, der die Assistenzfunktionalität in Bezug auf ein unbestimmtes der zwei oder mehr Objekten (231, 232, 233) anfordert.

9. Computer-implementiertes Verfahren nach einem der voranstehenden Ansprüche,
wobei die Bewegungsinformation einen optischen Fluss zwischen aufeinanderfolgenden Bildern der Abfolge von Bildern umfasst.

10. Computer-implementiertes Verfahren nach einem der voranstehenden Ansprüche,
wobei die Priorisierungsinformation mit einer Positionierung der zwei oder mehreren Objekten verknüpft ist.

11. Computer-implementiertes Verfahren nach einem der voranstehenden Ansprüche,
wobei die Priorisierungsinformation zumindest eines eine Segmentierungskarte mit Priorisierungslabel oder eine Punktlokalisation mit Priorisierungslabel umfasst.

12. Computer-implementiertes Verfahren nach einem der voranstehenden Ansprüche, wobei das Ausführen der Assistenzfunktionalität umfasst:
- Bestimmen einer Soll-Konfiguration des Operationsmikroskopiesystems (80) basierend auf der Priorisierungsinformation und einer Positionierung der zwei oder mehr Objekten (231, 232, 233), und
- Ansteuern von mindestens einer Komponente des Operationsmikroskopiesystems basierend auf der Soll-Konfiguration.

13. Computer-implementiertes Verfahren Anspruch 12,
wobei die Soll-Konfiguration des Operationsmikroskopiesystems eine Ausrichtung eines Gesichtsfelds (122, 123) des Mikroskops (84) in Bezug auf mindestens eines der zwei oder mehr Objekten (231, 232, 233), das in Abhängigkeit von der Priorisierungsinformation ausgewählt ist, umfasst.

14. Computer-implementiertes Verfahren nach Anspruch 12 oder 13,
wobei die Soll-Konfiguration des Operationsmikroskopiesystems eine Auto-Fokussierung des Mikroskops (84) in Bezug auf eines der zwei oder mehr Objekten (231, 232, 233), das in Abhängigkeit von der Priorisierungsinformation ausgewählt ist, umfasst.

15. Datenverarbeitungseinrichtung (60) zur Steuerung eines Operationsmikroskopiesystems (80), wobei die Datenverarbeitungseinrichtung (60) einen Prozessor (61) umfasst, der eingerichtet ist, um Programmcode aus einem Speicher (62) laden und ausführen kann, wobei das Ausführen des Programmcodes bewirkt, dass der Prozessor (61) die folgenden Schritte ausführt:
- Ansteuern (3105) einer Kamera (83, 86) des Operationsmikroskopiesystems zum Erhalten einer Abfolge von Bildern,
- Bestimmen (3115) von Bewegungsinformation für jedes von zwei oder mehr in der Abfolge der Bilder abgebildeten Objekten (78, 231, 232, 233) basierend auf der Abfolge von Bildern,
- Bestimmen (3120) von Priorisierungsinformation für die zwei oder mehr Objekte (78, 231, 232, 233) basierend auf der Bewegungsinformation, und
- basierend auf der Priorisierungsinformation, Ausführen (3125) einer Assistenzfunktionalität im Zusammenhang mit den zwei oder mehr Objekten (78, 231, 232, 233).
